# EUROPÄISCHE PATENTSCHRIFT

(11) **EP 0 840 594 B1**
(45) Veröffentlichungstag und Bekanntmachung des Hinweises auf die Patenterteilung: **23.01.2002**
(21) Anmeldenummer: 96927018.0
(22) Anmeldetag: 16.07.1996
(51) Int. Cl.: A61K 7/13

(54) **OXIDATIONSFÄRBEMITTEL ZUM FÄRBEN VON KERATINFASERN, DIE EINE SPEZIELLE KOMBINATION VON ENTWICKLERKOMPONENTEN UND ALKALISIERUNGSMITTEL ENTHALTEN**
OXIDATION COLORANTS FOR DYEING KERATINIC FIBERS, CONTAINING A SPECIAL COMBINATION OF DEVELOPER COMPONENTS AND ALKALISATION AGENTS
COLORANTS D'OXYDATION POUR COLORER DES FIBRES KERATINIQUES, CONTENANT UNE COMBINAISON SPECIALE DE COMPOSANTS DEVELOPPEURS ET D'AGENTS D'ALCALISATION

(30) Priorität: 25.07.1995 DE 19527124
(43) Veröffentlichungstag der Anmeldung: 13.05.1998
(73) Patentinhaber: Henkel Kommanditgesellschaft auf Aktien, 40191 Düsseldorf (DE)
(72) Erfinder: HÖFFKES, Horst, D-40595 Düsseldorf (DE); SCHRADER, Dieter, D-40589 Düsseldorf (DE); SCHOLZE, Barbara, D-40589 Düsseldorf (DE)
(86) Internationale Anmeldenummer: EP9603120
(87) Internationale Veröffentlichungsnummer: WO9704740

(56) Entgegenhaltungen:
- WO-A-93/19725
- DE-A- 2 215 303
- DE-A- 2 852 252
- DE-A- 3 929 333
- DE-A- 4 409 143

## Beschreibung

Die Erfindung betrifft Oxidationsfärbemittel zum Färben von Keratinfasern, die eine spezielle Kombination von Entwicklerkomponenten und Alkalisierungsmitteln enthalten.

Unter Keratinfasern sind dabei Pelze, Wolle, Federn und insbesondere menschliche Haare zu verstehen. Obwohl die erfindungsgemäßen Oxidationsfärbemittel in erster Linie zum Färben von Keratinfasern geeignet sind, steht prinzipiell einer Verwendung auch auf anderen Gebieten nichts entgegen.

Für das Färben von Keratinfasern, insbesondere menschlicher Haare, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und in den meisten Fällen Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Zur Erzielung optimaler Färbeergebnisse werden die Oxidationsfärbemittel üblicherweise alkalisch eingestellt. Die meisten kommerziellen Produkte weisen pH-Werte von 9,0 bis 10,5 auf. Zur Einstellung des pH-Wertes wurde bisher üblicherweise Ammoniak verwendet; weiterhin sind vor allem in jüngster Zeit Produkte auf den Markt gekommen, die als Alkalisierungsmittel kurzkettige Alkanolamine enthalten. Durch letztere wird zwar das Problem des Ammoniakgeruches deutlich verringert, dennoch erscheinen diese Amine noch nicht als optimale Alkalisierungsmittel. So kann es insbesondere bei häufiger Anwendung in höheren Konzentrationen zu ungewünschten Beeinträchtigungen der Haarstruktur kommen. Außerdem können höhere Konzentrationen an Alkanolaminen, insbesondere in Kombination mit Wasserstoffperoxid, bei sehr empfindlichen Personen in einzelnen Fällen Reizungen der Kopfhaut verursachen. Schließlich wird bei Verwendung von Ammoniak und kurzkettigen Aminen zur pH-Wert-Einstellung bei längerer Lagerung ein langsamer aber kontinuierlicher Abfall des pH-Wertes beobachtet.

Es hat daher nicht an Versuchen gemangelt, Oxidationsfärbemittel zu entwickeln, die neutral bis weniger stark alkalisch eingestellt sind. Hierbei tritt allerdings die große Schwierigkeit auf, daß viele Entwickler-Komponenten bei diesen pH-Werten nur unzureichend mit dem Oxidationsmittel reagieren. Davon sind insbesondere solche Komponenten betroffen, die zu Ausfärbungen im Rot-Gelb-Bereich führen. Die gleichzeitige Verwendung von direktziehenden roten oder gelben Farbstoffen kann dieses Problem nicht wirklich zufriedenstellend lösen. Es sei in diesem Zusammenhang nur auf die geringere Waschbeständigkeit der direktziehenden Farbstoffe hingewiesen.

In der DE-OS 22 15 303 wurde vorgeschlagen, Oxidationsfärbemittel für Haare mit einem Gehalt an Guanidin, Arginin oder deren Derivaten zu formulieren. Dadurch soll das Eindringen der Farbstoffvorläufer in den Haarschaft begünstigt und die Haarfarbstoffe stabilisiert werden.

Größere Mengen an Arginin oder ähnlichen Verbindungen führen aber häufig zu Problemen bei der Herstellung der Färbemitteln; insbesondere bei Mitteln auf Emulsionsbasis, wie sie auf dem Markt dominieren, wird häufig, vor allem bei längerer Lagerung, wie sie bei Verbraucherprodukten immer zu berücksichtigen ist, eine Phasentrennung beobachtet. Eine solche Änderung des Erscheinungsbildes des Produktes wird vom Konsumenten in der Regel mit einer Verschlechterung der Produkteigenschaften gleichgesetzt, weshalb solche Produkte auf dem Markt nicht plazierbar sind.

Es wurde nun überraschenderweise gefunden, daß die oben genannten Schwierigkeitkeiten überwunden werden können und Oxidationsfärbemittel mit ausgezeichneten Färbeeigenschaften formulierbar sind, wenn bestimmte Entwickler-komponenten eingesetzt werden und die Basizität des Färbemittels durch spezielle Alkalisierungsmittel eingestellt wird.

In einer speziellen Ausführungsform der Erfindung sind Färbemittel im praktisch "neutralen" pH-Bereich zugänglich, die auch ohne direktziehende Farbstoffe exzellente Färbeergebnisse liefern.

Gegenstand der Erfindung sind daher Oxidationsfärbemittel zum Färben von Keratinfasem, enthaltend Entwicklerkomponenten sowie gewünschtenfalls Kupplerkomponenten in einem wasserhaltigen Träger, die
- als Entwicklerkomponente ein Pyrimidin-Derivat, das 2 - 4 Aminosubstituenten und 0 - 2 Hydroxysubstituenten am Pyrimidin-Ring aufweist, und
- als Alkalisierungsmittel Aminosäuren oder Oligopeptide mit mindestens einer Aminogruppe und mindestens einer -COOH- oder -SO₃H-Gruppe, deren 2,5-%ige Lösung in Wasser einen pH-Wert von größer 9,0 aufweist,
enthalten.

In den erfindungsgemäßen Oxidationsfärbemitteln werden als Entwicklerkomponente Pyrimidin-Derivate mit 2 - 4 Aminosubstituenten und 0 - 2 Hydroxysubstituenten am Pyrimidin-Ring verwendet.

Die erfindungsgemäß verwendeten Pyrimidin-Derivate, wie beispielsweise 2,4,5,6-Tetraaminopyrimidin, 2-Hydroxy-4,5,6-triaminopyrimidin, 4-Hydroxy-2,5,6,-triaminopyrimidin und 2,4-Dihydroxy-5,6-diaminopyrimidin, sind dem Fachmann bekannte Verbindungen. Stellvertretend für eine Vielzahl von Druckschriften, die diese Verbindungen offenbaren, wird auf die DE-OS 23 59 399 sowie die Monographie von Kh. Schrader, Grundlagen und Rezepturen der Kosmetika, Dr. Alfred Hüthig Verlag, Heidelberg, 2. Auflage, verwiesen. Insbesondere auf den Inhalt der DE-OS 23 59 399, soweit er die Herstellung der Verbindungen offenbart, wird ausdrücklich Bezug genommen.

2-Hydroxy-4,5,6-triaminopyrimidin und 2,4,5,6-Tetraaminopyrimidin sind erfindungsgemäß bevorzugte Entwickler-Komponenten. Insbesondere führt die Verwendung des 2,4,5,6-Tetraaminopyrimidins zu hervorragenden Ergebnissen.

Die erfindungsgemäßen Oxidationsfärbemittel enthalten spezielle Alkalisierungsmittel.

Bevorzugte Alkalisierungsmittel sind Aminocarbonsäuren, insbesondere α-Aminocarbonsäuren und omega-Aminocarbonsäuren. Unter den α-Aminocarbonsäuren sind wiederum Lysin und insbesondere Arginin besonders bevorzugt

Die Aminosäuren können den erfindungsgemäßen Mitteln bevorzugt in freier Form zugegeben werden. In einer Reihe von Fällen ist es jedoch auch möglich, die Aminosäuren in Salzform einzusetzen. Bevorzugte Salze sind dann die Verbindungen mit Halogenwasserstoffsäuren, insbesondere die Hydrochloride und die Hydrobromide.

Weiterhin können die Aminosäuren auch in Form von Oligopeptiden und Proteinhydrotysaten eingesetzt werden, wenn sichergestellt ist, daß die erforderlichen Mengen der erfindungsgemäß eingesetzten Aminosäuren darin enthalten sind. In diesem Zusammenhang wird auf die Offenbarung der DE-OS 22 15 303 verwiesen, auf die ausdrücklich Bezug genommen wird.

Ein besonders bevorzugtes Alkalisierungsmittel ist Arginin, insbesondere in freier Form, aber auch als Hydrochlorid eingesetzt.

Das Alkalisierungsmittel ist in den erfindungsgemäßen Oxidationsfärbemitteln bevorzugt in Mengen von 0,5 bis 6 Gew.-%, insbesondere von 2 bis 6 Gew.-%, bezogen auf das gesamte Mittel, enthalten.

Der pH-Wert der erfindungsgemäßen Oxidationsfärbemittel wird bevorzugt auf einen Wert von 6,5 bis 10,5 eingestellt. Gemäß einer speziellen Ausführungsform sind pH-Werte von 6,5 bis 8,5 bevorzugt.

Neben den beiden genannten zwingenden Komponenten und Wasser können die erfindungsgemäßen Oxidationsfärbemittel alle für solche Mittel bekannten Inhaltsstoffe enthalten.

Dies sind insbesondere sogenannte Kupplerkomponenten, wie sie zur Erzielung bestimmter Nuancen üblicherweise eingesetzt werden.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 4-Chlor-resorcin, 3-Amino-2-chlor-6-methylphenol und 2-Methylresorcin.

Resorcin, 2-Methylresorcin und 4-Chlorresorcin sind für die erfindungsgemäßen Oxidationsfärbemittel besonders geeignete Kupplerkomponenten.

Neben den bevorzugt eingesetzten Entwickler- und gegebenenfalls Kuppler-Komponenten können die erfindungsgemäßen Oxidationsfärbemittel noch weitere Entwickler- und gegebenenfalls Kuppler-Komponenten enthalten, wenn ganz bestimmte Farbnuancen erzielt werden sollen.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone und 4-Aminopyrazolonderivate eingesetzt.

Spezielle Vertreter sind beispielsweise p-Toluylendiamin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5 und 4-Amino-3-methylphenol.

Die erfindungsgemäßen Haarfärbemittel enthalten sowohl die Entwicklerkomponenten als auch gegebenenfalls die Kupplerkomponenten bevorzugt in einer Menge von 0,01 bis 20 Gew.-%, vorzugsweise 0,5 bis 5 Gew.-%, jeweils bezogen auf das gesamte Oxidationsfärbemittel.

Werden sowohl Entwicklerkomponenten als auch Kupplerkomponenten eingesetzt, so geschieht dies im allgemeinen in etwa molaren Mengen zueinander. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1 : 0,5 bis 1 : 2 enthalten sein können.

Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide einzusetzen.

Durch die erfindungsgemäße Wahl der Entwickler- und Alkalisierungskomponenten sind praktisch alle Farbtöne durch alleinige Verwendung von Oxidationsfarbstoffen zugänglich. Es ist daher nur noch in wenigen Ausnahmefällen notwendig, zur weiteren Modifizierung der Farbnuancen neben den Oxidationsfarbstoffvorprodukten zusätzlich übliche direktziehende Farbstoffe, z.B. aus der Gruppe der Nitrophenylendiamine, Nitroaminophenole, Anthrachinone oder Indophenole, wie z.B. die unter den internationalen Bezeichnungen bzw. Handelsnamen HC Yellow 2, HC Yellow 4, Basic Yellow 57, Disperse Orange 3, HC Red 3, HC Red BN, Basic Red 76, HC Blue 2, Nitroblau, Disperse Blue 3, Basic Blue 99, HC Violet 1, Disperse Violet 1, Disperse Violet 4, Disperse Black 9, Basic Brown 16, Pikraminsäure und Rodol 9 R, bekannten Verbindungen, in einer Menge von 0,01 bis 20 Gew.-%, bezogen auf das gesamte Oxidationshaarfärbemittel zuzusetzen.

4-Amino-2-nitro-diphenylamin-2'-carbonsäure oder 6-Nitro-1,2,3,4-tetrahydrochinoxalin sind erfindungsgemäß besonders bevorzugte direktziehende Farbstoffe.

Es ist nicht erforderlich, daß die Oxidationsfarbstoffvorprodukte oder die fakultativ enthaltenen direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Zur Herstellung der erfindungsgemäßen Färbemittel werden die Oxidationsfarbstoffvorprodukte in einen geeigneten wasserhaltigen Träger eingearbeitet. Zum Zwecke der Haarfärbung sind solche Träger z.B. Cremes, Emulsionen, Gele oder auch tensidhaltige schäumende Lösungen, z.B. Shampoos, Schaumaerosole oder andere Zubereitungen, für die Anwendung auf dem Haar geeignet.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. in vielen Fällen hat es sich aber als vorteilhaft erwiesen, die Tenside aus anionischen, zwitterionischen oder nichtionischen Tensiden auszuwählen. Anionische Tenside können dabei ganz besonders bevorzugt sein.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ether-, Amid- und Hydroxylgruppen sowie in der Regel auch Estergruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare und verzweigte Fettsäuren mit 8 bis 22 C-Atomen (Seifen).
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobemsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungs produkte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C8-C22-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acylaminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈-C₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkyl-amidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester,
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Coming 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{R}-Quat 3270 und 3272 (Hersteller: Th. Goldschmidt; diquaternäre Polydimethylsiloxane, Quatemium-80).

Alkylamidoamine, insbesondere Fettsäureamidoamine wie das unter der Bezeichnung Tego Amid^{R}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quatemäre Esterverbindungen, sogenannte "Esterquats", wie die unter dem Warenzeichen Stepantex^{R} vertriebenen Dialkylammoniummethosulfate und Methyl-hydroxyalkyl-dialkoyloxyalkyl-ammoniummethosulfate.

Ein Beispiel für ein als kationisches Tensid einsetzbares quatemäres Zuckerderivat stellt das Handelsprodukt Glucquat^{R}100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide,
- hydroxide oder -alkoholate als Katalysatoren verwendet werden.

Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quatemisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quatemierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert.Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert. Butylacrylamid-Terpolymere,
- Verdickungsmittel wie Agar-Agar, Guar-Gum, Alginate, Xanthan-Gum, Gummi arabicum, Karaya-Gummi, Johannisbrotkemmehl, Leinsamengummen, Dextrane, Cellulose-Derivate, z. B. Methyicellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Glucose, Maleinsäure und Milchsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Farbstoffe zum Einfärben der Zubereitungen,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft,
- Antioxidantien
   sowie
- weitere Alkalisierungsmittel wie beispielsweise Ammoniak, Monoethanolamin, 2-Amino-2-methylpropanol und 2-Amino-2-methyl-propandiol-1,3.

Dabei werden weitere Alkalisierungsmittel bevorzugt nur in untergeordneten Mengen, d.h. in Mengen bis maximal 50 Gew.-%, insbesondere unter 20 Gew.-%, bezogen auf die Alkalisierungwsmittel gemäß Anspruch 1, eingesetzt.

Die Bestandteile des wasserhaltigen Trägers werden zur Herstellung der erfindungsgemäßen Färbemittel in für diesen Zweck üblichen Mengen eingesetzt; z.B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen insbesondere Wasserstoffperoxid oder dessen Anlagerungsprodukte an Harnstoff, Melamin oder Natriumborat in Frage. Auch können, insbesondere im neutralen bis schwächer alkalischen Bereich von pH 6,5 bis 8,5, Peroxydisulfate und -phosphate wie Kalium- und Ammoniumperoxydisulfat bzw. Dikaliumdihydrogenperoxydiphosphat eingesetzt werden.

Weiterhin ist es möglich, die Oxidation mit Hilfe von Enzymen durchzuführen. Dabei können die Enzyme sowohl zur Erzeugung von oxidierenden Per-Verbindungen eingesetzt werden als auch zur Verstärkung der Wirkung einer geringen Menge vorhandener oder enzymatisch erzeugter Oxidationsmittel. Ein Beispiel für ein enzymatisches Verfahren stellt das Vorgehen dar, die Wirkung geringer Mengen (z.B. 1 % und weniger, bezogen auf das gesamte Mittel) Wasserstoffperoxid durch Peroxidasen zu verstärken.

Zweckmäßigerweise wird die Zubereitung des Oxidationsmittels unmittelbar vor dem Haarefärben mit der Zubereitung aus den Oxidationsfarbstoffvorprodukten vermischt. Das dabei entstehende gebrauchsfertige Haarfärbepräparat sollte bevorzugt einen pH-Wert im Bereich von 8 bis 10 aufweisen. Besonders bevorzugt ist die Anwendung der Haarfärbemittel in einem schwach alkalischen Milieu. Die Anwendungstemperaturen können im Bereich der Kopfhaut oder wenig höher liegen. Nach einer Einwirkungszeit von ca. 30 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Weiterhin Gegenstand der Erfindung ist die Verwendung eines erfindungsgemäßen Mittels zur Färbung von Keratinfasern, insbesondere menschlichen Haaren.

Schließlich umfaßt die Erfindung auch ein Verfahren zum Färben von Keratinfasem, insbesondere menschlichen Haaren, unter Verwendung eines erfindungsgemäßen Mittels.

Das nachfolgende Beispiel soll den Erfindungsgegenstand näher erläutern.

### Beispiel

Es wurden ein Oxidationsfärbemittel mit der folgenden Zusammensetzung hergestellt (alle Mengenangaben sind Massen-%).

| Komponenten: | |
|---|---|
| | |
| Texapon^{R} N 28¹ | 20,0 |
| Dehyton^{R} K² | 4,0 |
| Fettalkoholgemisch C12-C18 | 10,0 |
| L-Arginin | 4,0 |
| EDTA, di-Na-Salz | 0,2 |
| Ammoniumdihydrogenphosphat | 0,6 |
| Natriumsulfit | 0,43 |
| Ascorbinsäure | 0,4 |
| Tetraaminopyrimidinsulfat | 0,32 |
| p-Aminophenol | 0,4 |
| p-Toluylendiaminsulfat | 0,16 |
| 2,7-Dihydroxynaphthalin | 0,08 |
| 2-Methylresorcin | 0,31 |
| Parfümöl | q.s. |
| Wasser | ad 100 |

| | |
|---|---|
| ¹ Natriumlaurylethersulfat (ca. 28 % Aktivsubstanz; INCI-Bezeichnung: Sodium Laureth Sulfate) (HENKEL) | |
| ² Fettsäureamid-Derivat mit Betainstruktur der Formel R-CONH(CH₂)₃N⁺(CH₃)₂CH₂COO⁻ (ca. 30 % Aktivsubstanz; INCI-Bezeichnung: Cocoamidopropyl Betaine) (HENKEL) | |

Die Creme wies einen pH-Wert von ca. 8,8 auf. Nach dem Vermischen mit einem polyacrylathaltigen Entwickler (enthaltend 3 % H₂O₂; pH-Wert: ca. 3,5) im Verhältnis 5:4 resultierte eine gebrauchsfertige Färbemischung mit einem pH-Wert von ca. 7,5.

Die Ausfärbung von hellblonden Haarsträhnen mit dieser Färbemischung ergab einen ausdrucksvollen Mahagoniton.

## Patentansprüche

1. Oxidationsfärbemittel zum Färben von Keratinfasem, enthaltend Entwicklerkomponenten und gewünschtenfalls Kupplerkomponenten in einem wasserhaltigen Träger, **dadurch gekennzeichnet, daß**
- als Entwicklerkomponente ein Pyrimidin-Derivat, das 2 - 4 Aminosubstituenten n und 0 - 2 Hydroxysubstituenten am Pyrimidin-Ring aufweist und
- als Alkalisierungsmittel eine Aminosäure oder Oligopeptid mit mindestens einer Aminogruppe und mindestens einer -COOH- oder -SO₃H-Gruppe, deren 2,5-%ige Lösung in Wasser einen pH-Wert von größer 9,0 aufweist, enthalten ist.

2. Oxidationsfärbemittel nach Anspruch 1, **dadurch gekennzeichnet, daß** das Pyrimidin-Derivat ausgewählt ist aus 2-Hydroxy-4,5,6-triaminopyrimidin und 2,4,5,6-Tetraaminopyrimidin

3. Oxidationsfärbemittel nach Anspruch 2, **dadurch gekennzeichnet, daß** das Pyrimidin-Derivat 2,4,5,6-Tetraaminopyrimidin ist

4. Oxidationsfärbemittel nach einem der Ansprüche 1 bis 3, **dadurch gekennzeichnet, daß** das Alkalisierungsmittel ausgewählt ist aus α-Aminosäuren.

5. Oxidationsfärbemittel nach Anspruch 4, **dadurch gekennzeichnet, daß** das Alkalisierungsmittel Arginin ist.

6. Oxidationsfärbemittel nach einem der Ansprüche 1 bis 5, dadurch ge kennzeichnet, daß mindestens eine Kupplerkomponente enthalten ist, ausgewählt aus der Gruppe, die von Resorcin, 2-Methylresorcin und 4-Chlorresorcin gebildet wird.

7. Oxidationsfärbemittel nach einem der Ansprüche 1 bis 6, dadurch ge kennzeichnet, daß Entwicklerkomponenten in einer Menge von 0,01 bis 20 Gew.-%, vorzugsweise 0,5 bis 5 Gew.-%, und gegebenenfalls Kupplerkomponenten in einer Menge von 0,01 bis 20 Gew.-%, vorzugsweise 0,5 bis 5 Gew.-%, jeweils bezogen auf das gesamte Färbemittel, enthalten sind.

8. Oxidationsfärbemittel nach einem der Ansprüche 1 bis 7, dadurch ge kennzeichnet, daß es einen pH-Wert von 6,5 bis 8,5 aufweist.

9. Oxidationsfärbemittel nach einem der Ansprüche 1 bis 8, **dadurch gekennzeichnet, daß** mindestens ein direktziehender Farbstoff enthalten ist.

10. Oxidationsfärbemittel nach Anspruch 9, **dadurch gekennzeichnet, daß** der direktziehende Farbstoff 4-Amino-2-nitro-diphenylamin-2'-carbonsäure oder 6-Nitro-1,2,3,4-tetrahydrochinoxalin ist.

11. Oxidationsfärbemittel nach einem der Ansprüche 1 bis 10, **dadurch gekennzeichnet, daß** es ein anionisches Tensid enthält.

12. Oxidationsfärbemittel nach Anspruch 11, **dadurch gekennzeichnet, daß** das anionische Tensid ein Salz einer gesättigten oder ungesättigten C8-C22-Carbonsäure ist.

13. Verwendung eines Mittels nach einem der Ansprüche 1 bis 12 zur Färbung von Keratinfasem, insbesondere menschlichen Haaren.

14. Verfahren zur Färbung von Keratinfasem, insbesondere menschlichen Haaren, **dadurch gekennzeichnet, daß** ein Mittel gemäß Ansprüchen 1 bis 12 auf das Haar aufgebracht und nach einer Einwirkzeit wieder abgespült wird

## Claims

1. An oxidation colorant for colouring keratin fibers containing primary intermediates and, if desired, secondary intermediates in a water-containing carrier, **characterized in that** they contain
- a pyrimidine derivative with 2 to 4 amino substituents and 0 to 2 hydroxy substituents on the pyrimidine ring as the primary intermediate and
- an amino acid or oligopeptide containing at least one amino group and at least one -COOH or -SO₃H group, of which a 2.5% solution in water has a pH value above 9.0, as the alkalizing agent.

2. An oxidation colorant as claimed in claim 1, **characterized in that** the pyrimidine derivative is selected from 2-hydroxy-4,5,6-triaminopyrimidine and 2,4,5,6-tetraaminopyrimidine.

3. An oxidation colorant as claimed in claim 2, **characterized in that** the pyrimidine derivative is 2,4,5,6-tetraaminopyrimidine.

4. An oxidation colorant as claimed in any of claims 1 to 3, **characterized in that** the alkalizing agent is selected from α-amino acids.

5. An oxidation colorant as claimed in claim 4, **characterized in that** the alkalizing agent is arginine.

6. An oxidation colorant as claimed in any of claims 1 to 5, **characterized in that** at least one secondary intermediate selected from the group consisting of resorcinol, 2-methyl resorcinol and 4-chlororesorcinol is present.

7. An oxidation colorant as claimed in any of claims 1 to 6, **characterized in that** primary intermediates are present in a quantity of 0.01 to 20% by weight and preferably 0.5 to 5% by weight while secondary intermediates are optionally present in a quantity of 0.01 to 20% by weight and preferably 0.5 to 5% by weight, based on the colorant as a whole.

8. An oxidation colorant as claimed in any of claims 1 to 7, **characterized in that** it has a pH value of 6.5 to 8.5.

9. An oxidation colorant as claimed in any of claims 1 to 8, **characterized in that** at least one substantive dye is present.

10. An oxidation colorant as claimed in claim 9, **characterized in that** the substantive dye is 4-amino-2-nitrodiphenylamine-2'-carboxylic acid or 6-nitro-1 ,2,3,4-tetrahydroquinoxaline.

11. An oxidation colorant as claimed in any of claims 1 to 10, **characterized in that** it contains an anionic surfactant.

12. An oxidation colorant as claimed in claim 11, **characterized in that** the anionic surfactant is a salt of a saturated or unsaturated C₈₋₂₂ carboxylic acid.

13. The use of the oxidation colorant claimed in any of claims 1 to 12 for colouring keratin fibres, more particularly human hair.

14. A process for colouring keratin fibres, more particularly human hair, **characterized in that** the hair colorant claimed in claims 1 to 12 is applied to the hair and, after a contact time, is rinsed off again.

## Revendications

1. Colorant par oxydation pour la coloration de fibres kératiniques, contenant des composants d'agent de développement et si désiré des composants d'agent de couplage, dans un véhicule contenant de l'eau,
**caractérisé en ce qu'**
Il contient :
- comme composant d'agent de développement, un dérivé de pyrimidine, qui possède 2 à 4 substituants amino et de 0 à 2 substituants hydroxy sur le cycle pyrimidine, et
- comme agent d'alcalinisation un acide aminé ou un oligopeptide ayant au moins un groupe aminé et au moins un groupe -COOH ou - SO₃H dont la solution à 2,5 % dans l'eau manifeste une valeur de pH supérieure à 9,0.

2. Colorant par oxydation selon la revendication 1,
**caractérisé en ce que**
le dérivé de pyrimidine est choisi parmi la 2-hydroxy-4,5,6-triaminopyrimidine et la 2,4,5,6-tétraaminopyrimidine.

3. Colorant par oxydation selon la revendication 2,
**caractérisé en ce que**
le dérivé de pyrimidine est la 2,4,5,6-tétraaminopyrimidine.

4. Colorant par oxydation selon l'une quelconque des revendications 1 à 3,
**caractérisé en ce que**
l'agent d'alcalinisation est choisi parmi les acides α-aminés.

5. Colorant par oxydation selon la revendication 4,
**caractérisé en ce que**
l'agent d'alcalinisation est l'arginine.

6. Colorant par oxydation selon l'une quelconque des revendications 1 à 5,
**caractérisé en ce qu'**
au moins un composant d'agent de couplage est contenu, choisi dans le groupe formé du résorcinol, du 2-méthylrésorcinol. et du 4-chlororésorcinol.

7. Colorant par oxydation selon l'une quelconque des revendications 1 à 6,
**caractérisé en ce que**
des composants d'agent de développement sont contenus en une quantité allant de 0,01 à 20 % en poids, de préférence de 0,5 à 5 % en poids et éventuellement des composants d'agent de couplage en une quantité allant de 0,01 à 20 % en poids de préférence de 0,5 à 5 % en poids, à chaque fois rapporté au colorant total.

8. Colorant par oxydation selon l'une quelconque des revendications 1 à 7,
**caractérisé en ce qu'**
il possède une valeur de pH allant de 6,5 à 8,5.

9. Colorant par oxydation selon l'une quelconque des revendications 1 à 8,
**caractérisé en ce qu'**
il contient au moins un colorant par action directe.

10. Colorant par oxydation selon la revendication 9,
**caractérisé en ce que**
le colorant à action directe est l'acide 4-amino-2-nitrodiphénylamine-2'-carboxylique, ou la 6-nitro-1,2,3,4-tétrahydroquinoxaline.

11. Colorant par oxydation selon l'une quelconque des revendications 1 à 10,
**caractérisé en ce qu'**
il contient un agent tensioactif anionique.

12. Colorant par oxydation selon la revendication 11,
**caractérisé en ce que**
l'agent tensioactif anionique est un sel d'un acide carboxylique saturé ou non saturé, en C₈ à C₂₂.

13. Utilisation d'une composition selon l'une quelconque des revendications 1 à 12 pour la coloration des fibres kératiniques, en particulier de cheveux humains.

14. Procédé de coloration de fibres kératiniques en particulier de cheveux humains,
**caractérisé en ce qu'**
un colorant de composition conforme aux revendications 1 à 12 est appliqué sur les cheveux et est rincé à nouveau après un temps d'action.
